# EUROPEAN PATENT SPECIFICATION

(11) **EP 3 538 887 B1**
(45) Date of publication and mention of the grant of the patent: **03.03.2021**
(21) Application number: 17801617.6
(22) Date of filing: 02.11.2017
(51) Int. Cl.: G01N 33/49

(54) **METHOD AND DEVICE FOR THROMBOCYTE COUNTING IN CAPILLARY BLOOD**
VERFAHREN UND VORRICHTUNG ZUR THROMBOZYTENZÄHLUNG IN KAPILLARBLUT
PROCÉDÉ ET DISPOSITIF DE COMPTAGE DE THROMBOCYTES DANS LE SANG CAPILLAIRE

(30) Priority: 11.11.2016 SE 1651481
(43) Date of publication of application: 18.09.2019
(73) Proprietor: Boule Medical AB, 163 53 Spånga (SE)
(72) Inventor: SELIN, Mariel, 182 76 Stocksund (SE); JOHANSSON, Mats, 111 36 Stockholm (SE); BAGGE, Jonas, 168 68 Bromma (SE)
(74) Representative: Ehrner & Delmar Patentbyrå AB
(86) International application number: PCT/EP2017/077998
(87) International publication number: WO 2018/086974

(56) References cited:
- US-A1- 2014 017 719
- US-B1- 6 284 548
- US-B2- 8 927 228
- U. HASSAN ET AL: "A microfluidic biochip for complete blood cell counts at the point-of-care", TECHNOLOGY, vol. 03, no. 04, 1 December 2015 (2015-12-01), pages 201-213, XP055434288, ISSN: 2339-5478, DOI: 10.1142/S2339547815500090

## Description

### TECHNICAL FIELD

The present invention relates to the field of analysis of capillary blood samples using automated devices, in particular to determination of thrombocyte counts from such samples.

### BACKGROUND TO THE INVENTION

Methods and devices for determining blood cell counts from capillary blood samples in a point-of-care setting are well known. In a typical case, a small sample (microliters) of capillary blood (as opposed to venous blood) is drawn into a thin glass capillary coated with an anticoagulant, typically EDTA. The glass capillary with the blood sample is then placed in an automated device, which performs a dilution in an isotonic saline buffer followed by determination of cell counts, such as red blood cells, various classes of white blood cells and thrombocytes (also called platelets).

In the technical field, it is a recognized problem that when using capillary blood samples to determine the thrombocyte count from a patient using an automated integrated instrument, the results are often falsely low compared to counts obtained using venous samples from the same patient, sampled around the same time point. The problem stems from the tendency of thrombocytes to aggregate despite use of powerful anticoagulants such as EDTA coated on the capillary walls.

A proposed solution to the problem is described in US 8,927,228, where the inventors have discovered that addition of chloroquine salts to the sample alleviates the problem of aggregates.

A distinct separate problem, which is not the aim of the present invention is EDTA-dependent pseudothrombocytopenia (PTCP), the rare phenomenon of a spurious low platelet counts due to EDTA-induced aggregation of platelets in some patients, which can be resolved e.g. with kanamycin. The unusual PTCP phenomenon is distinct from the general case of less accurate counts from capillary samples compared to venous samples.

However, there is still need in the field to provide alternative and/or improved methods and devices for thrombocyte determination from capillary blood using integrated automated devices, in particular obviating the need to use additional reagents and being cost-effective to implement.

### DEFINITIONS

The term *EDTA* refers to ethylenediaminetetraacetic acid, a chelating agent that binds calcium and other divalent metal cations.

The terms *thrombocyte* and *platelet* are used interchangeably.

The term *capillary blood* refers to a blood sample from a capillary of a subject, typically a puncture/stick of a finger. Capillary blood is distinct from venous blood, which is defined by being sampled from a vein, as well as from arterial blood sampled from an artery. In addition to the source, the sampling methods differ between capillary blood and venous blood, leading to distinct properties for samples of each category.

The term *MPA* refers to an adapter device designed for and used in the Medonic M-series M32 instruments and the Swelab Alfa Plus instruments. It is intended to receive and then dilute a small defined volume of a blood sample contained in a capillary tube open in both ends. The technology is described in US6284548B1.

### BRIEF DESCRIPTION OF THE FIGURES

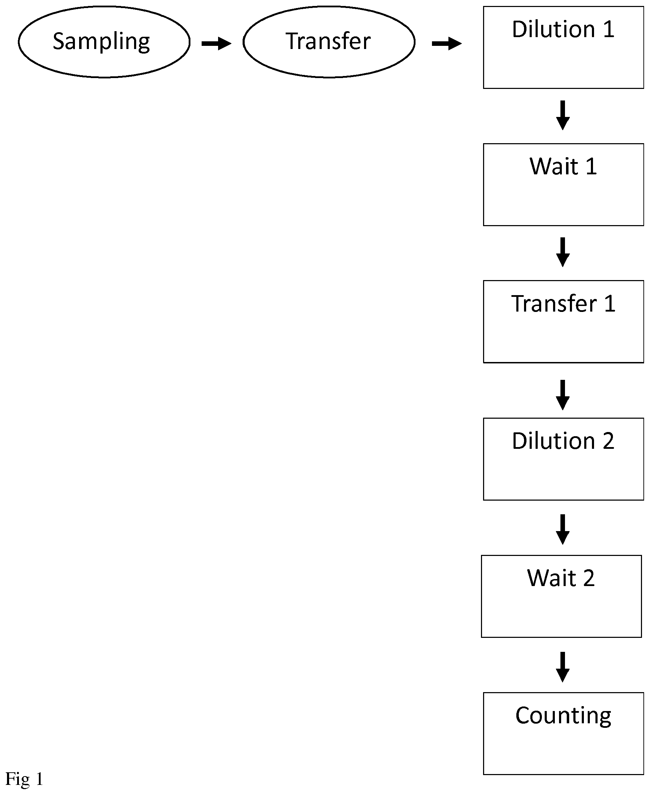

**Figure 1****:** Flow chart of a typical automated platelet counting procedure, exemplified as the procedure used in the Medonic M-series M32M and the Swelab Alfa Plus Standard. The ovals symbolize manual handling of the sample. The rectangles are automatic processing by the cell counter. **Sampling:** 20µl of blood is gathered via capillary action into the capillary tube (micro pipette). **Transfer:** Sample is put inside the MPA device. **Dilution 1:** Sample is moved to the mixing cup by flushing with diluent thereby creating the first dilution (ca. 4 s). **Wait 1:** In the mixing cup there is a short wait for the mixture to become homogenous (ca. 10 s in standard protocol. It is in this that the extra time has been added to stabilize the PLT results. **Transfer 1:** The first dilution is moved to a so-called shear valve that separates 20 µl of this dilution (ca. 3 s). **Dilution 2:** The sample is moved to the counting chamber by flushing with diluent thereby creating a second dilution (ca. 4 s). **Wait 2:** In the counting chamber, there is a short wait for the mixture to become homogenous (ca. 7 s). **Counting:** The cells in the sample are counted and classified via the Coulter principle (ca. 13 s).

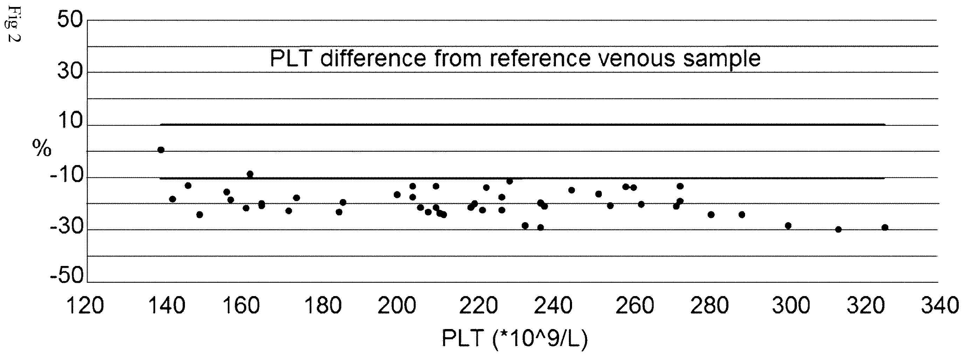

**Figure 2****:** Large clinical study showing relative differences between platelet counts from capillary blood using **standard procedure** compared to venous blood analysis.

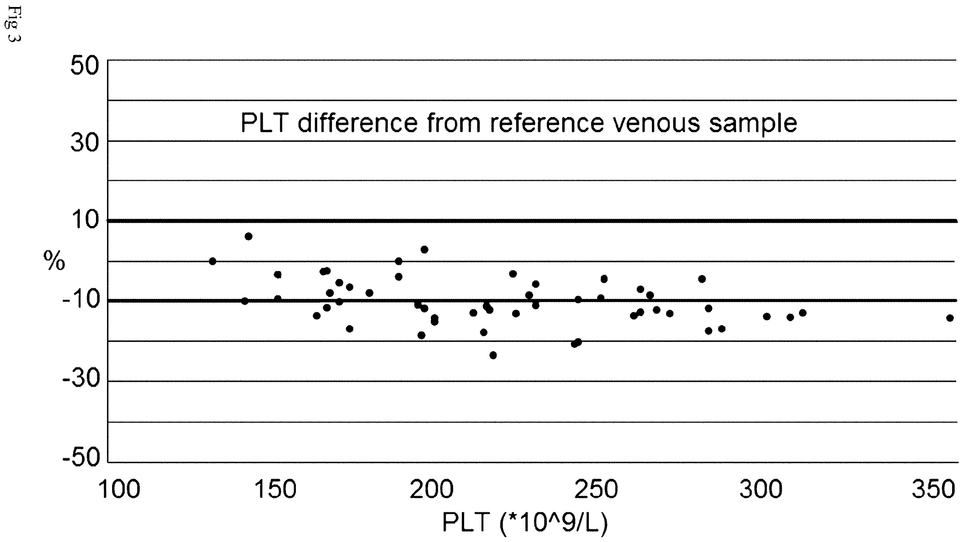

**Figure 3****:** Large clinical study showing relative differences between platelet counts from capillary blood using **inventive procedure,** having added 15 s incubation time (only to capillary counting procedure), compared to venous blood analysis.

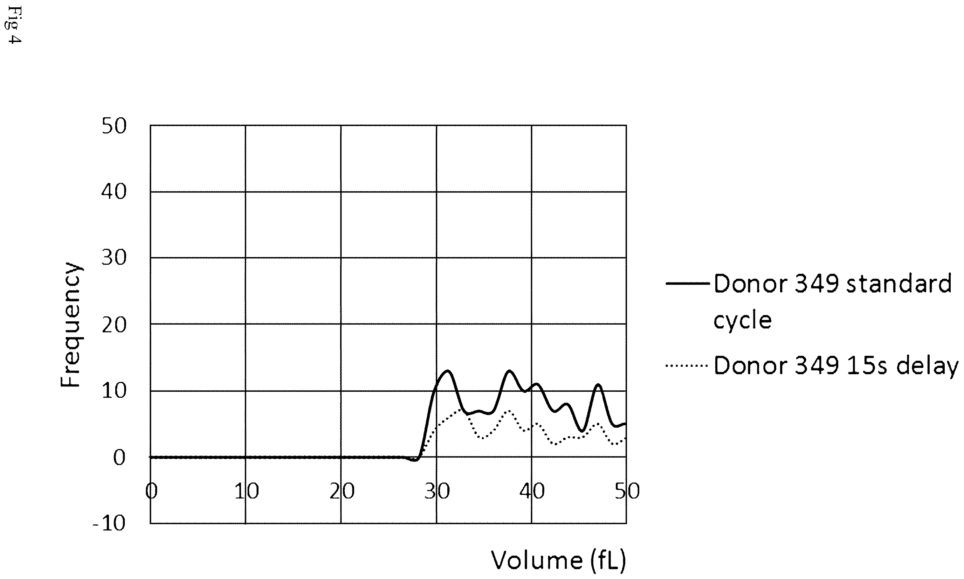

**Figure 4****:** WBC histograms for Donor 349 finger stick samples, standard MPA cycle vs. 15s delay in mixing chamber. Illustrative particle count graph from an individual donor showing minor difference between standard and inventive procedures. There is little apparent platelet aggregation to begin with, so there is little difference between the procedures.

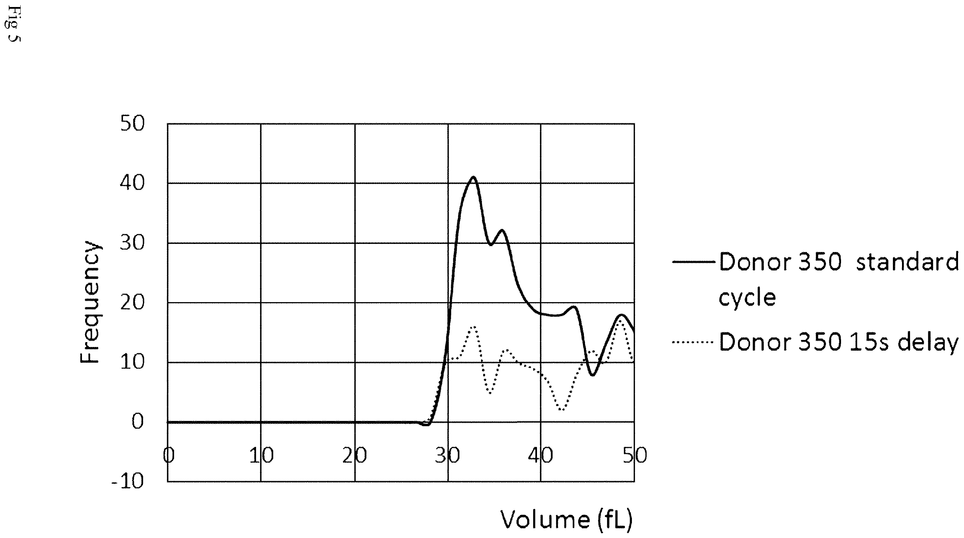

**Figure 5****:** WBC histograms for Donor 350 finger stick samples, standard MPA cycle vs. 15s delay in mixing chamber. Illustrative particle count graph from an individual donor showing a substantial difference between standard and inventive procedures. There is significant platelet aggregation using the standard procedure, but this is substantially mitigated by the inventive procedure.

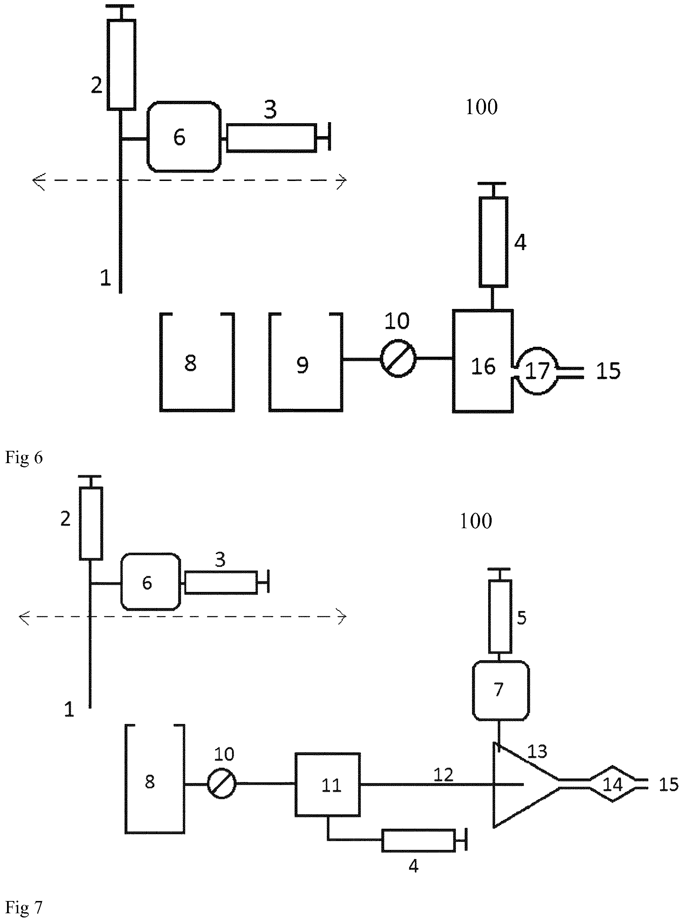

**Figures 6-9****:** Principle schematics and operations of automated integrated devices for determining cell counts in a blood sample.

### SUMMARY OF THE INVENTION

The present invention relates to the following items. The subject matter disclosed in the items below should be regarded disclosed in the same manner as if the subject matter were disclosed in patent claims.

In a first aspect, the present invention provides, a method for determining the thrombocyte count in a capillary blood sample, comprising the steps of:
a. providing a capillary blood sample subjected to anticoagulant treatment with EDTA at sample collection;
b. diluting said sample by a dilution factor of 1:10 to 1:2000, in a non-lytic buffer;
c. incubating the diluted sample for a duration of at least 23-300 s in the presence of an amount of EDTA efficient for reducing platelet aggregation, wherein the amount of EDTA is at least 0.1 mM;
d. optionally, diluting the incubated sample in a second dilution step prior to determining the thrombocyte count from the incubated sample; and
e. determining the thrombocyte count from the incubated sample;
   wherein the steps b, c, e and optionally d are performed in an integrated device for thrombocyte counting (100).

In an embodiment of the method according the first aspect, the capillary blood sample provided is a blood sample collected in an EDTA-coated sampling device.

In an embodiment of the method according the first aspect, the sampling device is an EDTA-coated capillary.

In an embodiment of the method according the first aspect, the dilution factor is 1:30 to 1:1000.

In an embodiment of the method according the first aspect, the dilution factor is 1:50 to 1:450.

In an embodiment of the method according the first aspect, the dilution factor is 1:150 to 1:300.

In an embodiment of the method according the first aspect, the dilution factor is 1:200 to 1:250.

In an embodiment of the method according the first aspect, the dilution factor is 1:225.

In an embodiment of the method according the first aspect, the incubated sample is subjected to a second dilution step prior to determining the thrombocyte count from the incubated sample.

In an embodiment of the method according the first aspect, the second dilution step involves diluting the sample by a second dilution factor of 1:50-1:1000.

In an embodiment of the method according the first aspect, the second dilution step involves diluting the sample by a second dilution factor of 1:100-1:300.

In an embodiment of the method according the first aspect, the second dilution step involves diluting the sample by a second dilution factor of 1:175-1:225.

In an embodiment of the method according the first aspect, the second dilution step involves diluting the sample by a second dilution factor of 1:200.

In an embodiment of the method according the first aspect, the non-lytic buffer is a buffered physiological saline solution, such as phosphate buffered saline or HEPES-buffered saline.

In an embodiment of the method according the first aspect, the incubation takes place in the presence of at least 0.1 mM EDTA.

In an embodiment of the method according the first aspect, the incubation takes place in the presence of at least 0.2 mM EDTA.

In an embodiment of the method according the first aspect, the incubation takes place in the presence of at least 0.3 mM EDTA.

In an embodiment of the method according the first aspect, the incubation takes place in the presence of at least 0.4 mM EDTA.

In an embodiment of the method according the first aspect, the incubation takes place in the presence of at least 0.5 mM EDTA.

In an embodiment of the method according the first aspect, the incubation takes place in the presence of 0.2-4 mM EDTA.

In an embodiment of the method according the first aspect, the incubation takes place in the presence of 0.3-4 mM EDTA.

In an embodiment of the method according the first aspect, the incubation takes place in the presence of 0.3-0.7 mM EDTA.

In an embodiment of the method according the first aspect, the incubation takes place in the presence of 0.1-5 mM EDTA.

In an embodiment of the method according the first aspect, the incubation takes place in the presence of 0.2-4 mM EDTA.

In an embodiment of the method according the first aspect, the incubation takes place in the presence of 0.3-3 mM EDTA.

In an embodiment of the method according the first aspect, the incubation takes place in the presence of 0.3-2 mM EDTA.

In an embodiment of the method according the first aspect, the incubation takes place in the presence of 0.3-1 mM EDTA.

In an embodiment of the method according the first aspect, the incubation takes place in the presence of 0.4-0.7 mM EDTA.

In an embodiment of the method according the first aspect, the incubation takes place in the presence of 0.4-0.6 mM EDTA.

In an embodiment of the method according the first aspect, the incubation takes place in the presence of 0.50-0.56 mM EDTA.

In an embodiment of the method according the first aspect, the incubation step duration is 25-90s.

In an embodiment of the method according the first aspect, the incubation step duration is 27-60 s.

In an embodiment of the method according the first aspect, the incubation step duration is 28-40 s.

In an embodiment of the method according the first aspect, the incubation step duration is 30-36 s.

In an embodiment of the method according the first aspect, the incubation step duration is 33 s.

In an embodiment of the method according the first aspect, the sample is not from a patient afflicted with EDTA-dependent pseudothrombocytopenia.

In an embodiment of the method according the first aspect, the steps b, c, e and optionally d are performed in a device according to any of the following items.

In a first aspect, the present invention provides, a device for determining the thrombocyte count in a capillary blood sample (100), comprising:
f. a receiver (1, 19) for a sampling device containing a capillary blood sample subjected to anticoagulant treatment with EDTA at sample collection;
g. a dilution element (8) for diluting said sample in a non-lytic buffer; and
h. a detector (14,17) for determining the thrombocyte count from the diluted sample;
   characterized in that the device is configured such that during operation:
   i. the sample is diluted in the dilution element (8) by a dilution factor of 1:10 to 1:2000; and
   ii. the diluted sample is incubated for a duration of 23-300 s prior to determination of the thrombocyte count from the diluted sample in the detector (14,17).

In an embodiment of the device according the second aspect, the incubation duration is 25-90s.

In an embodiment of the device according the second aspect, the incubation duration is 27-60 s.

In an embodiment of the device according the second aspect, the incubation duration is 28-40 s.

In an embodiment of the device according the second aspect, the incubation duration is 30-36 s.

In an embodiment of the device according the second aspect, the incubation duration is 33 s.

In an embodiment of the device according the second aspect, the dilution factor is 1:30 to 1:1000.

In an embodiment of the device according the second aspect, the dilution factor is 1:50 to 1:450.

In an embodiment of the device according the second aspect, the dilution factor is 1:150 to 1:300.

In an embodiment of the device according the second aspect, the dilution factor is 1:200 to 1:250.

In an embodiment of the device according the second aspect, the dilution factor is 1:225.

In an embodiment of the device according the second aspect, comprising a second and/or further dilution element(s) (9,13,16) arranged, during operation, for subjecting the sample to a second dilution step prior to determining the thrombocyte count from the incubated sample.

In an embodiment of the device according the second aspect, the second dilution step involves diluting the sample by a second dilution factor of 1:50-1:1000.

In an embodiment of the device according the second aspect, the second dilution step involves diluting the sample by a second dilution factor of 1:100-1:300.

In an embodiment of the device according the second aspect, the second dilution step involves diluting the sample by a second dilution factor of 1:175-1:225.

In an embodiment of the device according the second aspect, the second dilution step involves diluting the sample by a second dilution factor of 1:200.

In an embodiment of the device according the second aspect, the sampling device is an EDTA-coated capillary.

### DETAILED DESCRIPTION

Normally, the determination of thrombocyte counts from capillary blood involves a dilution step prior to determination. Furthermore, in case of automated analysis using integrated instruments, the determination after the dilution is performed in the shortest possible time, no more than a few seconds, to optimise instrument throughput. However, the known protocols used in such instruments lead to falsely low thrombocyte counts due to thrombocyte aggregation, unless additional reagents such as chloroquine salts are added to the diluted sample.

The inventors have unexpectedly found that the problematic aggregation of thrombocytes in capillary blood samples can be reversed by incubating a diluted capillary blood sample for a short time period (23 s or more), under certain conditions, before the thrombocyte counting is performed (see Examples 1-5).

The discovery provides a method for thrombocyte counting from capillary blood samples, which gives more accurate results without need for any additional reagents, such as chloroquine salts. Thus, existing reagents and kits can be used in the improved method with more accurate results, which is of great practical importance in many settings. Furthermore, in many instances, existing automated integrated devices for thrombocyte counting can be adapted to the more accurate method by a simple software update or other minor modification, whereby the inventive method is cost-effective to implement.

### Method for determining thrombocyte count

In a first aspect, the present invention provides a method for determining the thrombocyte count in a capillary blood sample, comprising the steps of:
a. providing a capillary blood sample subjected to anticoagulant treatment with EDTA at sample collection;
b. diluting said sample by a dilution factor of 1:10 to 1:2000, in a non-lytic buffer;
c. incubating the diluted sample for a duration of at least 23-300 s in the presence of an amount of EDTA efficient for reducing platelet aggregation, wherein the amount of EDTA is at least 0.1 mM;
d. optionally, diluting the incubated sample in a second dilution step prior to determining the thrombocyte count from the incubated sample; and
e. determining the thrombocyte count from the incubated sample.

That the capillary blood sample is subjected to anticoagulant treatment with EDTA at sample collection means that the blood sample is brought into contact with EDTA immediately after being sampled, preferably within less than 1s, more preferably the sample is collected into an EDTA-containing container. The anticoagulant treatment necessarily implies that the blood sample is subjected to an amount of EDTA sufficient for inhibiting coagulation.

The capillary blood sample provided may be a blood sample collected in an EDTA-coated sampling device containing an amount of EDTA sufficient to have a coagulation-inhibiting effect on the sample. The sampling device may be an EDTA-coated capillary.

The non-lytic buffer may be a buffered physiological saline solution, such as phosphate buffered saline or HEPES-buffered saline. Many different known types of buffers may be used, as long as they do not damage or change the thrombocytes to be counted to such an extend as to impede the counting.

In accordance with the invention, the dilution, incubation and determination steps (b, c and e), as well as the optional second dilution step (d) if present, are performed using an automated integrated device for thrombocyte counting (100) (optionally including readout on other blood parameters as well), such as a device of the second aspect described below. Adding an extra delay for incubation according to the present invention in integrated devices runs counter to conventional design principles, since such devices are normally designed to perform the analysis in the shortest possible time to optimise performance.

Incubations of the EDTA-coated capillary in which the blood sample was taken prior to dilution steps was also tested (see Table II in Example 3) for 2 minutes, 6 minutes and 10 minutes as compared to 0 minutes, i.e. with all these 4 incubations before dilution then following the normal analytical cycle. The outcome was that thrombocyte count showed an increase up to the 6 minutes and then stabilized. An incubation before dilution thus seems to be able to give similar results as the method of the present invention, but at a much slower rate. The improvements seen after a 6-minute incubation of the undiluted sample are equivalent to only a few seconds incubation after dilution. Given that rapid results and high throughput are highly desirable, the method of the present invention provides a significant improvement compared to the option of simply incubating the samples in the capillaries.

Preferably, the sample is not from a patient afflicted with EDTA-dependent pseudothrombocytopenia, a rare phenomenon than can be resolved e.g. using kanamycin.

### Dilution and EDTA concentration

As discussed above and shown under Examples 3 and 5, the platelet counts also can be improved by an incubation before dilution occurs. However, this is much less effective from a time performance perspective, and incubation at a dilution 1:45000 was not effective at all. Therefore, the incubated sample needs to be diluted to within a certain range (1:10 to 1:2000) during incubation for optimal results. Preferably, the dilution factor is 1:30 to 1:1000, more preferably 1:50 to 1:450, yet more preferably 1:150 to 1:300, still more preferably 1:175 to 1:225 and most preferably 1:225.

As shown in Example 4, the incubation must take place in the presence of an amount of EDTA efficient for reducing platelet aggregation to achieve improved results. What constitutes an efficient EDTA concentration in each specific case depends on other components in the non-lytic buffer, since EDTA has the effect of binding divalent metal cations in solution. The binding of Ca²⁺, thus reducing the concentration of free Ca²⁺ is generally considered the main mechanism for anticoagulant action of EDTA. While not wishing to be bound by theory, it is theorized that the divalent cation (in particular Ca²⁺) binding activity is behind the observed effect of incubation on platelet aggregation. Several classes of cell adhesion molecules relevant for platelet aggregation, such as integrins and cadherins are Ca²⁺ dependent so aggregation mediated by such cell adhesion molecules would be inhibited by an EDTA concentration sufficient for lowering the level of free Ca²⁺ below a certain threshold.

It is apparent from the above reasoning, that a higher concentration of Ca²⁺ ions in the diluent means that a higher concentration of EDTA is required, in order to achieve the sufficiently low concentration of free Ca²⁺ ions. Thus, the efficient amount of EDTA will depend on the concentration of divalent metal cations in the buffer. If other chelating agents that also bind Ca²⁺ are present, the required concentration of EDTA is lowered. Therefore, the effective concentration of EDTA needs to be determined in view of the other compounds present in the buffer. It is a matter of routine design and experimentation for a person having ordinary skill in the art to determine the effective EDTA concentration for the circumstances at hand, given the teachings herein.

Preferably, the incubation takes place in the presence of at least 0.1 mM EDTA, more preferably at least 0.2 mM EDTA, yet more preferably 0.3 mM EDTA, still more preferably at least 0.4 mM EDTA, most preferably at least 0.5 mM EDTA.

Also preferably, the incubation takes place in the presence of 0.2-4 mM EDTA, more preferably 0.3-4 mM EDTA, yet more preferably 0.3-0.7 mM EDTA.

The incubation may take place in the presence of 0.1-5 mM EDTA, preferably 0.2-4 mM EDTA, more preferably 0.3-3 mM EDTA, yet more preferably 0.3-2 mM EDTA, still more preferably 0.3-1 mM EDTA, even more preferably 0.4-0.7 mM EDTA, yet even more preferably 0.4-0.6 mM EDTA, most preferably 0.50-0.56 mM EDTA.

### Incubation time

In the present context, by incubation it is meant that the sample is within the appropriate effective limits of dilution and EDTA concentration irrespective of whether other activities (such as mixing) are simultaneously ongoing or not.

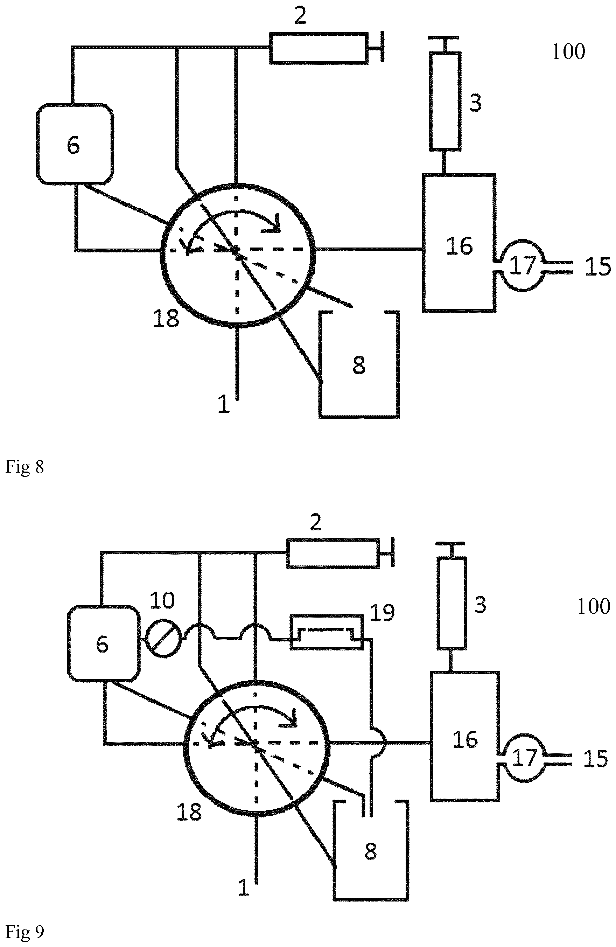

The principle is best understood when explained in the framework of a concrete example. As illustrated in a non-limiting fashion in Table A, a typical automated thrombocyte determination method comprises a number of handling steps, with a defined duration. In the case illustrated in Table A, the sample is placed in the instrument (such as an instrument of Fig 9) and the analysis process initiated. The sample to be analyzed is moved to a dilution element by flushing with diluent. The flushing step has a duration of 4 seconds, and as the end result 1:225 is achieved in 4 seconds, it can be assumed that it takes less than 1 second to achieve a first dilution in the range of the invention. The standard procedure calls for a 10 s mixing/incubation step to allow the first dilution to become homogenous. This incubation step is the most-straight-forward to prolong in the inventive method (see Table A). After incubation, an aliquot is taken from the first dilution. Duration the aliquot removal is 3 s in the case illustrated, and during that step the sample still has the same dilution factor of the first dilution, so this step can be considered part of the incubation in the context of the present inventive method.

The aliquot is then flushed with diluent to the counting chamber thus creating a second dilution. As the flushing duration is 4 s and the dilution factor is 1:225, it can be deduced that it takes 1 second at most to dilute the sample beyond the dilution factor specified in the claims. The second dilution is allowed to mix and cell counting performed in the detector.

Thus, the standard procedure can be regarded as incubating the sample within the appropriate limits of dilution and EDTA concentration for the invention for a total of 18 s, whereas the inventive method shown in comparison results in a total of 33 s incubation.

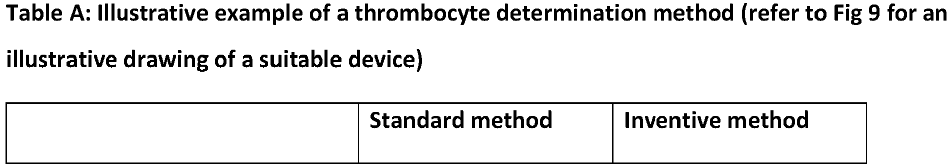

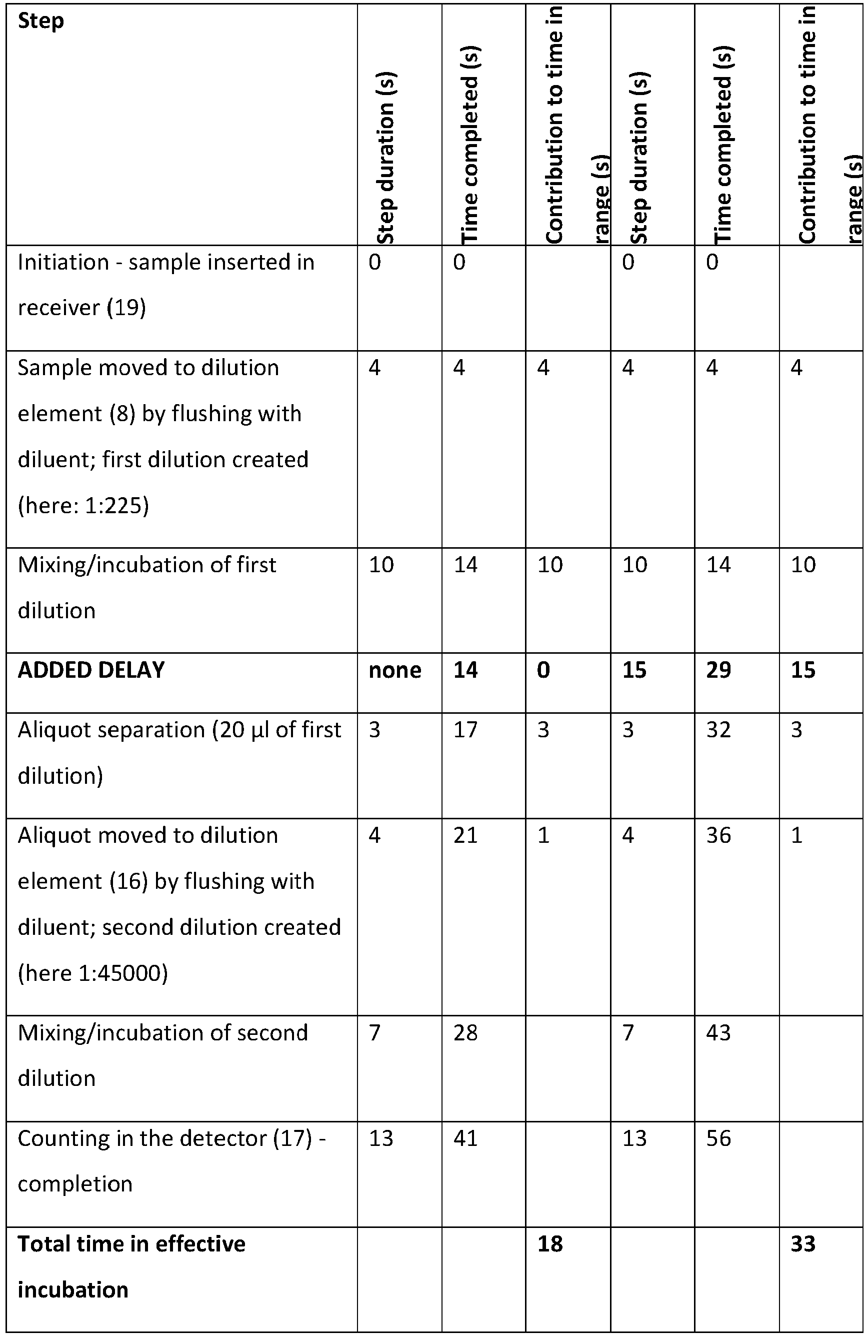

Example 3 was performed in an instrument that follows the standard cycle illustrated in Table A. As shown in Table I of Example 3, a significant improvement in platelet count can be discerned when an added time delay of 5s was added to the incubation step. From an added delay of 15s onwards the effect is better than at 5 s and remains the same for longer incubations. There does not appear to be any clear upper limit, but longer incubation periods than necessary are undesirable as they increase the analysis time and reduce throughput. An optimal incubation period seems to be about 33 s, since this incubation time results in stable, full effect on platelet counts in the shortest time.

Thus, the incubation step duration may be 23-300 s, preferably 25-90s, more preferably 27-60 s, yet more preferably 28-40 s, still more preferably 30-36 s and most preferably 33 s.

### Optional second dilution step

It may be desirable for practical reasons to perform a second dilution step subsequent to the incubation but prior to platelet count determination. Depending on the counting device (detector), additional dilution may be necessary for the determination to be feasible. In the case illustrated in Table A, there is a second dilution step with 1:200 dilution, resulting in a total dilution of 1:45000 before the platelet count is determined.

The second dilution step may involve diluting the sample by a second dilution factor of 1:50-1:1000, preferably 1:100-1:300, more preferably 1:175-1:225 and most preferably 1:200.

### Device determining thrombocyte count

Existing devices that are possible to configure to perform the method according to the first aspect of the present invention are known in the prior art and commercially available.

The absolute majority of haematology devices used for making a blood count use a system with two dilution steps. Such a system can readily be modified by addition of a receiver for a sampling device for a capillary blood sample and configured to perform the method of the first aspect of the present invention e.g. by a software update.

Detection of cells is usually achieved either via the "Coulter principle" or the "Flow cytometry principle". Both are well known methods, and both the methods are impaired by aggregation of the thrombocytes when determining the thrombocyte count.

The table below lists four representative example systems including the M-series M32 system used in the examples, using different means to achieve a two-step dilution and thrombocyte count, in an integrated and automated fashion. All the systems discussed here could readily be modified to perform the method as exemplified by the M-series M32 system, with guidance from the teachings herein.

The dilution elements and detectors for determining the cell count of red blood cells and thrombocytes employed in each instrument are shown in distilled schematic overviews (Figs 6-9).

| **Manufacturer** | **Device model name** | **Notes** |
|---|---|---|
| Abbot Diagnostics | Celldyn 1800 | Uses a pipette system with two dilution elements for the final dilution |
| Sysmex | XN-450 | Uses a pipette system with a bath and then a flow injection system for the final dilution |
| Diatron Group | Abacus 5 | Uses a shear valve with two baths for the final dilution. |
| Boule Medical | M-series M32 | Uses a shear valve with two baths for the final dilution. |

### Explanation of components in figs. 6-9

**1:** A receiver for a sampling device containing a capillary blood sample being a blood inlet for blood samples contained in vacutainers or microtainers. This component may also be adapted to receive a capillary tube containing a blood sample, see component 19.
**2-5:** Syringes for moving, pushing and pulling blood samples and diluent around in the device.
**6-7:** Containers for diluent. Diluent is a non-lytic buffer solution, and contains an efficient amount of EDTA for use with the present invention.
**8-9:** Dilution elements for diluting and mixing the blood sample with the non-lytic buffer solution.
**10:** Valve for opening or closing a pathway in the liquid system of the device.
**11:** Element for holding the sample while other operations such a closing a valve is taking place.
**12:** Injector needle for injecting a sample in the middle of another liquid flowing around it.
**13:** Hydrodynamic focusing chamber, contains a port in the backend where diluent is pushed into the chamber at a higher rate than the sample that is injected via the injector needle. This dilutes the sample per the different flow rates and via laminar flow the sample is focused exactly in the centre of the liquid stream exiting the chamber. This component can be regarded as a second dilution element.
**14:** A detector using a flow cytometer setup for determining the number of thrombocytes. A flow cytometer uses a laser focused in the middle of a stream of cells passing through the focus via an optically clear flow channel. The laser light scatters on the cells passing through the laser focus and analysis of the scattered lights strength and composition at different angles tells us the number of cells and types of cells that are passing through the focus.
**15:** Waste
**16:** A further dilution element for the final dilution and mixing of the sample with a non-lytic buffer solution.
**17:** A detector using the coulter principle setup for determining the number of thrombocytes. The coulter principle employs a small hole usually 60-80 µm in diameter and a positive electrode on one side and a negative electrode on the other side. The sample that is suspended in a conductive diluent is pushed through the small hole and when a cell passes through the change in resistance can be measured on the current passing between the electrodes. The change in resistance is proportional to the size of the cell and knowing the size of the cells it is possible to count and classify the cells.
**18:** Shear valve. This is a rotary valve that has a channel with a known volume that can be connected to different inlets and outlets. The shear valve operates by filling the channel with blood via one set of connecting ports after which it is rotated, shearing of a specific volume and connecting that volume to another set of connecting ports.
**19:** A receiver for a sampling device containing a capillary blood sample being a micro pipette adapter intended to receive a capillary tube open at both ends with a specified volume of capillary blood (see US6284548B1 for details). The diluent used for creating the first dilution with the blood from the blood inlet can be diverted via a normally closed valve to use the blood in the capillary tube instead.
**100:** An integrated device for thrombocyte counting, optionally with readout for other blood parameters.

### Pipette system with two baths principle schematic and operation for the count of thrombocytes (Fig 6).

Receiver shown as blood inlet 1 is in position to receive a blood sample. Syringe 2 pulls a specific volume of the blood sample into blood inlet **1.** Receiver blood inlet 1 is moved over the dilution element **8.** Syringe **3** ejects the sample with diluent **6** into the dilution element **8** to create the first dilution. Receiver blood inlet **1** is dipped into the dilution element **8** and syringe **2** pulls a specific volume of the first dilution into blood inlet **1.** Blood inlet **1** is moved over the second dilution element **9.** Syringe **3** ejects the sample with diluent **6** into the second dilution element **9** to create the second dilution. Valve **10** is opened and syringe **4** pulls the second and final dilution into third dilution element **16.** Valve **10** is closed and syringe **4** pushes the dilution through the detector **17** using the *coulter principle* to determine the thrombocyte cell count before finally being ejected as waste **15.**

### Pipette system with a bath and flow injection principle schematic and operation for the count of thrombocytes (Fig 7)

Receiver shown as blood inlet **1** is in position to receive a sample. Syringe **2** pulls a specific volume of the blood sample into receiver blood inlet **1.** The receiver blood inlet **1** is moved over the dilution element **8.** Syringe **3** ejects the sample together with diluent **6** into the dilution element **8** to create the first dilution. Valve **10** is opened and syringe **4** pulls the first dilution into holding element **11.** Valve **10** is closed and syringe **4** moves the first dilution via the injector needle **12** into the hydrodynamic focusing chamber **13,** at the same time syringe **5** moves diluent **7** into the hydrodynamic focusing chamber **13.** The ratio of dispensed diluent from syringe **5** and first dilution from syringe **4** defines the ratio of the second dilution of the sample as it is pushed through the detector **14** using the *flow cytometry principle* for the thrombocyte cell count and then finally to be ejected as waste **15.**

### Shear valve system with two baths principle schematic and operation for the count of thrombocytes (Fig 8)

Shear valve **18** connects the receiver shown as blood inlet **1** and syringe **2.** Syringe **2** pulls the blood sample via receiver blood inlet **1** into the shear valve **18.** The shear valve **18** connects diluent **6** and dilution element **8.** Syringe **2** pushes diluent **6** and the blood in the shear valve **18** into dilution element **8** creating the first dilution. Shear valve **18** connects syringe **2** and dilution element **8.** Syringe **2** pulls the first dilution into the shear valve **18.** Shear valve **18** connects diluent **6** and dilution element **16.** Syringe **2** pushes diluent **6** and the first dilution in shear valve **18** into dilution element **16** creating the second and final dilution. Shear valve **18** connects receiver blood inlet **1** and syringe **2,** closing the path to/from the dilution element. Syringe **3** pushes the second and final dilution through the detector **17** using the *coulter principle* to determine the thrombocyte cell count before finally being ejected as waste **15.**

### M-series M32M with a micro pipette adapter and a shear valve system with two baths principle schematic and operation for the count of thrombocytes (Fig 9)

Two different dilution operations can be performed.

If valve **10** is closed throughout the operation, then the procedure as described for fig. 8 can be performed.

If valve **10** is used to divert the diluent through the micro pipette adapter, then the following procedure can be performed. Refer to Table A above for durations of the various stages.

Shear valve **18** is closed to all connections. A capillary tube with a blood sample is inserted into the receiver, a micro pipette adapter **19.** Valve **10** is open and syringe **2** pushes diluent **6** through valve **10** and the blood in the receiver micro pipette adapter **19** into the dilution element **8** creating the first dilution. The device may be configured to perform the inventive incubation at this stage (see Table A). Valve **10** is closed.

### From this position the procedure continues as in Fig.8 with valve 10 closed.

Shear valve **18** connects syringe **2** and dilution element **8.** Syringe **2** pulls the first dilution into the shear valve **18.** Shear valve **18** connects diluent **6** and (second) dilution element **16.** Syringe **2** pushes diluent **6** and the first dilution in shear valve **18** into dilution element **16** creating the second and final dilution. Shear valve **18** connects blood inlet **1** and syringe **2,** closing the path to/from the dilution element. Syringe **3** pushes the second and final dilution through the detector **17** using the *coulter principle* to determine the thrombocyte cell count before finally being ejected as waste **15.**

All these haematology devices could either replace the first blood aspiration step with an adapter or use a parallel adapter, as exemplified by the M-series M32M, to serve as a receiver for a sampling device containing a capillary blood sample subjected to anticoagulant treatment with EDTA at sample collection. This adapter would be intended to receive and dilute a small defined volume of a blood sample contained in a capillary tube open in both ends (see US6284548B1 for an exemple of a suitable adapter).

Thus, in a second aspect, the present invention provides a device for determining the thrombocyte count in a capillary blood sample, comprising:
a. a receiver (1, 19) for a sampling device containing a capillary blood sample subjected to anticoagulant treatment with EDTA at sample collection;
b. a dilution element (8) for diluting said sample in a non-lytic buffer;
c. a detector (14, 17) for determining the thrombocyte count from the diluted sample;
   characterized in that the device is configured such that during operation:
   i. the sample is diluted in the dilution element (8) by a dilution factor of 1:10 to 1:2000; and
   ii. the diluted sample is incubated for a duration of at least 23 s prior to performing determination of the thrombocyte count in the detector (14, 17).

During operation, the device may be configured such that the incubation during operation takes place in the presence of an amount of EDTA efficient for reducing platelet aggregation. The non-lytic buffer used for dilution in the dilution element (8) during operation may have an EDTA concentration resulting in an effective EDTA concentration as specified under the disclosure of the first aspect.

The device may have a physical configuration as shown in Fig 9, with operational configuration per Fig 1 or Table A.

The device may be configured such that incubation duration is 23-300 s, preferably 25-90s, more preferably 27-60 s, yet more preferably 28-40 s, still more preferably 30-36 s and most preferably 33 s.

The device may be configured such that the dilution factor is 1:30 to 1:1000, preferably 1:50 to 1:400, more preferably 1:150 to 1:300, yet more preferably 1:200 to 1:250, most preferably 1:225.

The device may comprise a second and/or further dilution element(s) (9, 13, 16) arranged, during operation, for subjecting the sample to a second and/or further dilution step(s) prior to determining the thrombocyte count from the incubated sample, for example as shown in Figs 6, 7, 8 and 9.

The second dilution step may involve diluting the sample by a second dilution factor of 1:50-1:1000, preferably 1:100-1:300, more preferably 1:175-1:225 and most preferably 1:200.

### General aspects pertaining to the present disclosure

The term "comprising" is to be interpreted as including, but not being limited to. The arrangement of the present disclosure into sections with headings and subheadings is merely to improve legibility and is not to be interpreted limiting in any way, in particular, the division does not in any way preclude or limit combining features under different headings and subheadings with each other.

### EXAMPLES

For further experimental details, the skilled reader is directed to the section Material and Methods.

### Example 1: Prolonged post-dilution incubation time improves thrombocyte counting from capillary blood

It was serendipitously discovered during product development that increasing the incubation time after the first dilution step by 15s, the thrombocyte counts seemed to improve, in terms of being closer to the true values obtained from the same individuals using venous blood samples.

In an earlier data set (presented in Fig 2 as a comparative example) it was clearly apparent that in the vast majority of healthy volunteers, the thrombocyte counts obtained from capillary blood were 10-30% below the values obtained from venous blood. The effect seems to be more pronounced in individuals with higher thrombocyte counts (see data along X-axis).

The serendipitous discovery prompted the inventors to perform similar experiments but with the added time delay for incubation. It is apparent from Fig 3 that the added time delay consistently brought the capillary measurements closer to the values obtained from venous samples.

Figure 1 illustrates the flow of the actual protocols used.

### Example 2: Improved thrombocyte counts are due to reduced aggregation

Some individual donors exhibit marked differences between capillary and venous samples whereas others do not. A comparison of particle counts from an individual donor NOT exhibiting difference between capillary and venous counts shows that the added time delay does not markedly change the distribution of particle frequency counts (Fig 4).

In contrast, in an individual donor exhibiting large difference between capillary and venous count it can be seen that particle count in the 30-45 fL range is drastically reduced by the added 15 s time delay (Fig 5).

It is known from literature that platelet aggregates are detected in this range, so it was concluded that the added time delay improved the accuracy of thrombocyte counts by reducing platelet aggregation.

### Example 3: Effective incubation times

In order to explore the effect further, different added time delays were tested. From the results in table I it can be seen that an improvement can be discerned already with 5 s added delay. No further improvement is apparent for incubation times with more than 15 s added delay.

**Table I. Effect of different incubation times. Average PLT values from all donor fingerstick samples as a % of the reference venous sample**

| | *Added time delay for sample in dilution cup (s)* | | | | | | | |
|---|---|---|---|---|---|---|---|---|
| *Assay type* | +5 | +10 | +15 | +30 | +45 | +60 | +90 | +300 |
| Test measurement with delay as indicated | 91% (86-104% spread) | 85% (77-92% spread) | 97% (91-102% spread) | 94% (84-107% spread) | 95% (93-99% spread) | 95% (89-104% spread) | 94% (88-98% spread) | 97% |
| Paired measurements with standard protocol (no delay) | 87% (82-104% spread) | not done | 86% (81-97% spread) | 82% (73-86% spread) | 85% (82-92% spread) | 85% (71-94% spread) | 87% (72-94% spread) | Not done |

In another experiment, the effect of mixing was explored. The added delay was set to 60 s with mixing every 15 seconds. The results (Table II) indicated no further improvement compared to 15 s added delay.

**Table II: No additional effect from mixing. Average PLT values from all donor fingerstick samples as a % of the reference venous sample (two different Medonic instruments)**

| | *Test run 1* | *Test run 2* |
|---|---|---|
| **Standard** MPA (0+ s delay) | 86% (67-97% spread) | 92% (84-105% spread) |
| Added **time delay** (60+ s with mixing every 15s) | 97% (89-103% spread) | 96% (92-104% spread) |

It should be stressed that incubation of the sample in the capillary (i.e. before dilution) for several minutes results in a similar but much slower improvement effect on PLT counts as seen in Table III below.

**Table III Mean PLT values from 10 donors with 4 different incubation times tested before first dilution occurring.**

| Time (min) incubation | Mean PLT |
|---|---|
| 0 | 202 |
| 2 | 212 |
| 6 | 228 |
| 10 | 225 |

These results depict how the PLT does improve with time though at a much slower rate than when the incubation is performed after dilution. The time taken to reach the acceptable PLT value is not optimal or acceptable on the market.

### Example 4: Effective EDTA concentration is required, EDTA required at sampling

Since the standard commercial diluent used in the system contains a small amount of EDTA (0.53 mM), the inventors experimented whether a higher EDTA concentration would improve the results further (Table IV).

**Table IV: Sample collected in standard boule EDTA plastic microcapillaries, varying concentration of EDTA in diluent (Average PLT values from all donor fingerstick samples as a % of the reference venous sample)**

| | *Standard Boule EDTA plastic microcapillaries* | | | |
|---|---|---|---|---|
| | **5+ sec delay** | **10+ sec delay** | **15+ sec delay** | **300+ sec delay** |
| Diluent with **NO EDTA** (0g EDTA/20L) | - | | 88.4% | - |
| **Standard** diluent (4g EDTA/20L). EDTA at **0.53mM.** | 91% | - | 91.7% (experiment 1) 96.1% (experiment 2) | 99.5% (exp 1) |
| Diluent with **extra EDTA** (20g extra EDTA/20L). EDTA was **3.1mM.** | 83.7% | - | in second dilution 1:45000 84.6% | - |

It is to be noted that a certain amount of EDTA is carried over from the capillary with the sample. The "standard EDTA capillaries" are coated with 100 µg K₂EDTA and collects 20 µl of blood. The molar mass of K2EDTA is approximately 368 g/mol, so the capillary contains about 0.27 µmol of EDTA. The concentration in the capillary, assuming all EDTA is completely eluted is maximally to 13.5 mM. The standard dilution of 1:225 would consequently amount to 0.06 mM capillary-derived EDTA at most. Since using diluent with no EDTA was not efficient, it can be concluded that an efficient EDTA concentration should be higher than that, under the present conditions.

The standard dilution buffer used in the test system contains 0.53 mM EDTA, and was shown to be an effective amount. Additional EDTA at 3.1 mM did not seem result in any further benefits even when the incubation time was very short (5s).

In another experiment is was also noted that when the sample was diluted to 1:45000 in a diluent containing 3.1 mM EDTA, the effect on platelet count was absent. The effect of dilution ratios was further explored in Example 5.

### Example 4: Effect of anticoagulant presence at sample collection

For comparison, samples were collected in untreated capillaries and platelet counts performed. Interestingly, even in the presence of additional EDTA in diluent, the platelet counts were not improved by longer incubation (Table V). Collection in plain capillaries may cause irreversible effects on platelet aggregation.

**Table V: Plain glass microcapillaries without EDTA do not perform as well (Average PLT values from all donor fingerstick samples as a % of the reference venous sample)**

| **EDTA in diluent** | **15+ sec delay** | **300+ sec delay** |
|---|---|---|
| 0 | 78,7% | - |
| 0.53 mM (standard), experiment 1 | 76,4% | |
| 0.53 mM (standard), experiment 2 | 82,6% | 90,1% |
| 3.1 mM | 77,6% | - |

In yet another test, the results in plain capillaries (no-EDTA) were 7% lower than with EDTA capillaries using the standard cycle with no delays.

### Example 5: Effective dilution ratios

Since the effect was no longer present at 1:45000 dilution (see Example 3), and is not present without any dilution, experiments were conducted to compare different dilution ratios. Note that for practical reasons, the same donor could not be tested at more than 3 dilutions on the same occasion.

As shown in Table VI, experiments with dilutions at 1:50 to 1:450 were all effective. The lower dilutions (1:50 to 1:225) appear somewhat better in terms of spread than 1:450, where the effect seems to be somewhat less stable.

**Table VI: Comparisons between dilution ratios.**

| | Relative PLT value from various dilution ratios normalized to reference dilution 1:225 (set as 100) | | | | |
|---|---|---|---|---|---|
| Donor | **1:56 +15s time delay** | **1:112 +15s time delay** | **1:225 +15s time delay** | **1:450 +15s time delay** | |
| 1 | 101.1 | | 100 | 95.0 | Part 4 data |
| 2 | 101.3 | | 100 | 91.5 | |
| 3 | 98.2 | | 100 | 96.2 | |
| 4 | 98.9 | | 100 | 102.6 | |
| 5 | 96.9 | | 100 | 100.2 | |
| 6 | 103.6 | | 100 | 90.8 | Part 3 data |
| 7 | 100.4 | | 100 | 95.1 | |
| 8 | | 97.8 | 100 | 87.0 | Part 2 data |
| 9 | | 99.7 | 100 | 104.1 | Part 1 data |
| 10 | | 92.6 | 100 | 80.5 | |
| 11 | | 107.7 | 100 | 108.1 | |
| 12 | | 92.0 | 100 | 91.4 | |
| 13 | | 102.2 | 100 | 103.4 | |
| 14 | | | | | |
| **Averages:** | **100** | **99** | **100** | **96** | |
| Max | 103.6 | 107.7 | 100.0 | 108.1 | |
| Min | 96.9 | 92.0 | 100.0 | 80.5 | |
| Spread | 6.7 | 15.7 | 0.0 | 27.6 | |

### MATERIALS AND METHODS

### Instruments and calibration materials

Medonic M-series M32C SN100778, experimental fw v1.3j5 based on fw v1.3
Medonic M-series M32S SN100011, experimental fw v1.3j5 based on fw v1.3
Swelab Alfa Plus Standard SN100779, experimental fw v1.3j5 based on fw v1.3
Boule Con-Diff Normal Lot# 21605-72
Boule Con-Diff Normal Lot# 21608-72
Boule Calibrator Lot# 21606-34

### Capillaries

Boule microcapillaries, Lot# 15964507, exp. 2019-02
EDTA-free glass micro capillaries, Gmbh+, Lot# 1902542

### Other materials and reagents

K2-EDTA vacutainers Lot# A15013WK
Diluent Lot# 1606-599 was used for the various diluent-EDTA concentration studies
BD 2.0mm Lancets Lot# N8R1259

### Capillary sampling procedure

### Preparations

- Check that all material to be used is at hand
- Check that the donors chair has armrests
- Wash your hands with soap and water
- Disinfect and your hands with disinfection alcohol for and let hands dry in air.
- Wear protective gloves, lab coat and a plastic apron
- Ensure the donor is clear on the procedure

### Procedure

- Choose finger, if possible the middle or the ring-finger.
- The location for sampling should be either side of the most distal phalanx of the middle or ring finger, on the palm side.
- If the donor's hands are cold they should be warmed first, the donors hand should be warm and relaxed
- Disinfect the fingertip with an injection swab
- Let the alcohol dissipate in the air.
- Use a Contact-Activated Lancet, Blue, High flow, BD Diagnostics preferably
- Turn the donors palm downwards during the sample procedure.
- Place the lancet slightly on the location indicated above and activate it.
- Remove the lancet immediately from the finger so the blood can flow freely.
- Dispose of the lancet.
- Wipe of the first drop of blood.
- Wait for more blood and then let the micro capillary draw blood without touching the skin until it is filled.

Note: Never squeeze the finger hard, if the blood doesn't flow then warm the hand more and repeat the procedure.

### Thrombocyte determination procedure

- Ensure the instrument is functioning correctly as per the instructions in the user manual.
- Carefully wipe away any blood caught on the outside of the capillary.
- Remove the MPA-adapter from its holder in the instrument and insert the micro capillary into it.
- Reinsert the adapter into the instrument and the automatic measurement of the sample will be started.

Note: Refer to the user manual for a complete set of instructions for the instrument.

## Claims

1. A method for determining the thrombocyte count in a capillary blood sample, comprising the steps of:
a. providing a capillary blood sample subjected to anticoagulant treatment with EDTA at sample collection;
b. diluting said sample by a dilution factor of 1:10 to 1:2000, in a non-lytic buffer;
c. incubating the diluted sample for a duration of 23-300 s in the presence of an amount of EDTA efficient for reducing platelet aggregation, wherein the amount of EDTA is at least 0.1 mM;
d. optionally, diluting the incubated sample in a second dilution step prior to determining the thrombocyte count from the incubated sample; and
e. determining the thrombocyte count from the incubated sample;
wherein the steps b, c, e and optionally d are performed in an integrated device for thrombocyte counting (100).

2. The method according to claim 1, wherein the capillary blood sample provided is a blood sample collected in an EDTA-coated sampling device.

3. The method according to any of the preceding claims, wherein the dilution factor is 1:150 to 1:300.

4. The method according to any of the preceding claims, wherein the incubated sample is subjected to a second dilution step prior to determining the thrombocyte count from the incubated sample.

5. The method according to claim 4, wherein the second dilution step involves diluting the sample by a second dilution factor of 1:100-1:300.

6. The method according to any of the preceding claims, wherein the incubation takes place in the presence of 0.1-5 mM EDTA, preferably 0.50-0.56 mM EDTA.

7. The method according to any of the preceding claims, wherein the incubation step duration is 28-40 s.

8. The method according to any of the preceding claims, wherein the sample is not from a patient afflicted with EDTA-dependent pseudothrombocytopenia.

9. The method according to any of the preceding claims, wherein the steps b, c, e and optionally d are performed in a device according to any of the following claims.

10. An integrated device for determining the thrombocyte count in a capillary blood sample (100), comprising:
a. a receiver (1, 19) for a sampling device containing a capillary blood sample subjected to anticoagulant treatment with EDTA at sample collection;
b. a dilution element (8) for diluting said sample in a non-lytic buffer; and
c. a detector (14,17) for determining the thrombocyte count from the diluted sample;
**characterized in that** the device is configured such that during operation:
i. the sample is diluted in the dilution element (8) by a dilution factor of 1:10 to 1:2000; and
ii. the diluted sample is incubated for a duration of 23-300 s prior to determination of the thrombocyte count from the diluted sample in the detector (14,17).

11. The device according to any of the preceding device claims, wherein the incubation duration is 28-40 s.

12. The device according to any of the preceding device claims, wherein the dilution factor is 1:30 to 1:1000, preferably 1:150 to 1:300.

13. The device according to any of the preceding device claims, comprising a second and/or further dilution element(s) (9,13,16) arranged, during operation, for subjecting the sample to a second dilution step prior to determining the thrombocyte count from the incubated sample.

14. The device according to claim 13, wherein the second dilution step involves diluting the sample by a second dilution factor of 1:100-1:300.

15. The device according to any of the preceding device claims, wherein the sampling device is an EDTA-coated capillary.

## Patentansprüche

1. Verfahren zur Bestimmung der Thrombozytenzahl in einer Kapillarblutprobe, umfassend die Schritte:
a. Bereitstellen einer Kapillarblutprobe, die bei der Probenahme Antikoagulationsmittelbehandlung mit EDTA unterworfen worden ist;
b. Verdünnen der Probe um einen Verdünnungsfaktor von 1:10 bis 1:2000 in einem nichtlytischen Puffer;
c. Inkubieren der verdünnten Probe für eine Dauer von 23-300 s in Gegenwart einer Menge von EDTA, die wirksam ist, Plättchenaggregation zu verringern, wobei die Menge an EDTA wenigstens 0,1 mM beträgt;
d. gegebenenfalls Verdünnen der inkubierten Probe in einem zweiten Verdünnungsschritt vor der Bestimmung der Thrombozytenzahl aus der inkubierten Probe; und
e. Bestimmen der Thrombozytenzahl aus der inkubierten Probe;
wobei die Schritte b, c, e und gegebenenfalls d in einer integrierten Vorrichtung zur Thrombozytenzählung (100) durchgeführt werden.

2. Verfahren gemäß Anspruch 1, wobei die bereitgestellte Kapillarblutprobe eine in einer EDTAbeschichteten Probenahmevorrichtung gesammelte Blutprobe ist.

3. Verfahren gemäß einem der vorstehenden Ansprüche, wobei der Verdünnungsfaktor 1:150 bis 1:300 beträgt.

4. Verfahren gemäß einem der vorstehenden Ansprüche, wobei die inkubierte Probe einem zweiten Verdünnungsschritt vor der Bestimmung der Thrombozytenzahl aus der inkubierten Probe unterworfen wird.

5. Verfahren gemäß Anspruch 4, wobei der zweite Verdünnungsschritt Verdünnen der Probe um einen Verdünnungsfaktor von 1:100-1:300 umfasst.

6. Verfahren gemäß einem der vorstehenden Ansprüche, wobei die Inkubation in Gegenwart von 0,1-5 mM EDTA, vorzugsweise 0,50-0,56 mM EDTA, erfolgt.

7. Verfahren gemäß einem der vorstehenden Ansprüche, wobei die Dauer des Inkubationsschritts 28-40 s beträgt.

8. Verfahren gemäß einem der vorstehenden Ansprüche, wobei die Probe nicht von einem Patienten stammt, der von EDTA-abhängiger Pseudothrombozytopenie betroffen ist.

9. Verfahren gemäß einem der vorstehenden Ansprüche, wobei die Schritte b, c, e und gegebenenfalls d in einer Vorrichtung gemäß einem der nachstehenden Ansprüche durchgeführt werden.

10. Integrierte Vorrichtung zur Bestimmung der Thrombozytenzahl in einer Kapillarblutprobe (100), umfassend:
a. eine Aufnahme (1, 19) für eine Probenahmevorrichtung die eine Kapillarblutprobe enthält, die bei der Probenahme Antikoagulationsmittelbehandlung mit EDTA unterworfen worden ist;
b. ein Verdünnungselement (8) zum Verdünnen der Probe in einem nichtlytischen Puffer; und
c. einen Detektor (14, 17) zum Bestimmen der Thrombozytenzahl aus der verdünnten Probe;
**dadurch gekennzeichnet, dass** die Vorrichtung so gestaltet ist, dass während des Betriebs:
i. die Probe in dem Verdünnungselement (8) um einen Verdünnungsfaktor von 1:10 bis 1:2000 verdünnt wird; und
ii. die verdünnte Probe für eine Dauer von 23-300 s vor der Bestimmung der Thrombozytenzahl aus der verdünnten Probe in dem Detektor (14, 17) inkubiert wird.

11. Vorrichtung gemäß einem der vorstehenden Ansprüche, wobei die Inkubationsdauer 28-40 s beträgt.

12. Vorrichtung gemäß einem der vorstehenden Ansprüche, wobei der Verdünnungsfaktor 1:30 bis 1:1000, vorzugsweise 1:1500 bis 1:300, beträgt.

13. Vorrichtung gemäß einem der vorstehenden Ansprüche, umfassend ein zweites und/oder weitere Verdünnungselement(e) (9, 13, 16), dafür angeordnet, während des Betriebs die Probe einem zweiten Verdünnungsschritt vor der Bestimmung der Thrombozytenzahl aus der inkubierten Probe zu unterwerfen.

14. Vorrichtung gemäß Anspruch 13, wobei der zweite Verdünnungsschritt Verdünnen der Probe um einen Verdünnungsfaktor von 1:100-1:300 umfasst.

15. Vorrichtung gemäß einem der vorstehenden Vorrichtungsansprüche, wobei die Probenahmevorrichtung eine EDTA-beschichtete Kapillare ist.

## Revendications

1. Procédé permettant de déterminer le nombre de thrombocytes dans un échantillon de sang capillaire, comprenant les étapes suivantes :
a. fournir un échantillon de sang capillaire soumis à un traitement anticoagulant avec de l'EDTA au niveau d'un prélèvement d'échantillon ;
b. diluer ledit échantillon par un facteur de dilution de 1:10 à 1:2 000, dans une solution tampon non lytique ;
c. incuber l'échantillon dilué pendant une durée de 23 à 300 s en présence d'une quantité d'EDTA efficace pour réduire l'agrégation plaquettaire, dans lequel la quantité d'EDTA est d'au moins 0,1 mM ;
d. éventuellement, diluer l'échantillon incubé dans une seconde étape de dilution avant de déterminer le nombre de thrombocytes à partir de l'échantillon incubé ; et
e. déterminer le nombre de thrombocytes à partir de l'échantillon incubé ;
dans lequel les étapes b, c, e et éventuellement d sont réalisées dans un dispositif intégré pour la numération des thrombocytes (100).

2. Procédé selon la revendication 1, dans lequel l'échantillon de sang capillaire fourni est un échantillon de sang prélevé dans un dispositif d'échantillonnage revêtu d'EDTA.

3. Procédé selon l'une quelconque des revendications précédentes, dans lequel le facteur de dilution est de 1:150 à 1:300.

4. Procédé selon l'une quelconque des revendications précédentes, dans lequel l'échantillon incubé est soumis à une seconde étape de dilution avant de déterminer le nombre de thrombocytes à partir de l'échantillon incubé.

5. Procédé selon la revendication 4, dans lequel la seconde étape de dilution fait intervenir la dilution de l'échantillon par un second facteur de dilution de 1:100 à 1:300.

6. Procédé selon l'une quelconque des revendications précédentes, dans lequel l'incubation a lieu en présence de 0,1 à 5 mM d'EDTA, de préférence 0,50 à 0,56 mM d'EDTA.

7. Procédé selon l'une quelconque des revendications précédentes, dans lequel la durée de l'étape d'incubation est de 28 à 40 s.

8. Procédé selon l'une quelconque des revendications précédentes, dans lequel l'échantillon ne provient pas d'un patient atteint de pseudothrombopénie liée à l'EDTA.

9. Procédé selon l'une quelconque des revendications précédentes, dans lequel les étapes b, c, e et éventuellement d sont réalisées dans un dispositif selon l'une quelconque des revendications suivantes.

10. Dispositif intégré permettant de déterminer le nombre de thrombocytes dans un échantillon de sang capillaire (100), comprenant :
a. un récepteur (1, 19) pour un dispositif d'échantillonnage contenant un échantillon de sang capillaire soumis à un traitement anticoagulant avec de l'EDTA au niveau d'un prélèvement d'échantillon ;
b. un élément de dilution (8) pour diluer ledit échantillon dans une solution tampon non lytique ; et
c. un détecteur (14, 17) pour déterminer le nombre de thrombocytes à partir de l'échantillon dilué ;
**caractérisé en ce que** le dispositif est configuré de sorte que, pendant le fonctionnement :
i. l'échantillon soit dilué dans l'élément de dilution (8) par un facteur de dilution de 1:10 à 1:2 000 ; et
ii. l'échantillon de dilution soit mis à incuber pendant une durée de 23 à 300 s avant la détermination du nombre de thrombocytes à partir de l'échantillon dilution dans le détecteur (14, 17).

11. Dispositif selon l'une quelconque des revendications de dispositif précédentes, dans lequel la durée d'incubation est de 28 à 40 s.

12. Dispositif selon l'une quelconque des revendications de dispositif précédentes, dans lequel le facteur de dilution est de 1:30 à 1:1 000, de préférence de 1:150 à 1:300.

13. Dispositif selon l'une quelconque des revendications de dispositif précédentes, comprenant un second et/ou un autre élément de dilution (9, 13, 16) conçus, pendant le fonctionnement, pour soumettre l'échantillon à une seconde étape de dilution avant de déterminer le nombre de thrombocytes à partir de l'échantillon incubé.

14. Dispositif selon la revendication 13, dans lequel la seconde étape de dilution fait intervenir la dilution de l'échantillon par un second facteur de dilution de 1:100 à 1:300.

15. Dispositif selon l'une quelconque des revendications de dispositif précédentes, dans lequel le dispositif d'échantillonnage est un capillaire revêtu d'EDTA.
